# EUROPÄISCHE PATENTSCHRIFT

(11) **EP 3 763 228 B1**
(45) Veröffentlichungstag und Bekanntmachung des Hinweises auf die Patenterteilung: **03.09.2025**
(21) Anmeldenummer: 20184960.1
(22) Anmeldetag: 09.07.2020
(51) Int. Cl.: A23L 33/18, A23L 33/185, A23L 33/21, A23L 33/00, A61K 38/01, A23J 3/14

(54) **ZUSAMMENSETZUNG ENTHALTEND EINE KOMBINATION PFLANZLICHER PROTEINE UND PEPTIDE**
COMPOSITION COMPRISING A COMBINATION OF VEGETABLE PROTEINS AND PEPTIDES
COMPOSITION CONTENANT UNE COMBINAISON DE PROTÉINES VÉGÉTALES ET DE PEPTIDES

(30) Priorität: 11.07.2019 DE 202019103828 U; 16.08.2019 DE 102019212317
(43) Veröffentlichungstag der Anmeldung: 13.01.2021
(73) Patentinhaber: Quiris Healthcare GmbH & Co. KG, 33334 Gütersloh (DE)
(72) Erfinder: Lange, Peer, 33824 Werther (DE)
(74) Vertreter: BRP Renaud & Partner mbB Rechtsanwälte Patentanwälte Steuerberater

(56) Entgegenhaltungen:
- US-A- 5 547 927
- DATABASE GNPD [online] MINTEL; 10 September 2015 (2015-09-10), ANONYMOUS: "Carob Flavored Rice & Pea Protein", XP055750446, Database accession no. 3430081
- ANONYMOUS: "Amino acid dietary supplement tablets", GNPD, MINTEL, 1 August 2015 (2015-08-01), XP002769164
- ANONYMOUS: "Product Guide 2017", 1 January 2017 (2017-01-01), XP055751709, Retrieved from the Internet <URL:https://greenlineingredients.com/botanicals/> [retrieved on 20201118]

## Beschreibung

Die vorliegende Erfindung betrifft eine Zusammensetzung, insbesondere zur Verwendung als Nahrungsergänzungsmittel, als Lebensmittel des allgemeinen Verzehrs oder als funktionelles Lebensmittel, enthaltend die Komponenten Erbsenproteinisolat, Pflanzenprotein-Hydrolysat und gegebenenfalls mindestens eine weitere Komponente, ausgewählt aus einer Gruppe, zumindest umfassend Kürbiskernprotein, Haferkleie, Sanddornpulver und Acerolapulver. Insbesondere ist die erfindungsgemäße Zusammensetzung ein funktionelles Lebensmittel, ein Nahrungsergänzungsmittel oder ein Lebensmittel für besondere medizinische Zwecke (FSMP).

Mit steigendem Alter verändert sich bei den meisten Menschen auch der Stoffwechsel. Ab dem 25. Lebensjahr verliert man pro Jahr etwa 1 % Stoffwechselumsatz und 1 % Muskelmasse, daher kommt es häufig zu einer unerwünschten Gewichtszunahme bei gleichzeitigem Verlust von Muskelmasse. Auch die kalorienreichen und nährstoffarmen industriellen Lebensmittel gefährden unsere Gesundheit und führen nicht selten zu Übergewicht [Meier et al. Healthcare costs associated with an adequate intake of sugars, salt and saturated fat in Germany: A health econometrical analysis. PLoS ONE, 2015].

Etwa 60 % der Männer und 40 % der Frauen in Deutschland sind übergewichtig. Die ernährungs- und altersbedingten Stoffwechselstörungen sind häufig durch einen spezifischen Mikro- und Makronährstoffmangel bedingt [Ramos-Lopez et al. Guide for current nutrigenetic, nutrigenomic, and nutriepigenetic approaches for precision nutrition involving the prevention and management of chronic diseases associated with obesity. J Nutrigenet Nutrigenomics, 2017].

Viele Menschen wünschen sich ein natürlich, dauerhaft und ganzheitlich ausgerichtetes Gewichtsmanagement mit Stoffwechselsteuerung ohne Hunger und Stress. Sie wollen gesund altern, aktiv leben und fit bleiben. Dazu muss man dem ernährungs- und altersbedingten Übergewicht und Muskelschwund entgegenwirken [Kalinkovich et al. Sarcopenic obesity or obese sarcopenia: A cross talk between age-associated adipose tissue and skeletal muscle inflammation as a main mechanism of the pathogenesis. Ageing Res Rev, 2017].

Für beide Ziele ist eine proteinbetonte Ernährung zielführend. Im Markt befindliche Präparate zum Muskelaufbau, sowie Formula-Diäten enthalten meistens Milchproteine wie z.B. Molke ("Whey") oder Casein als Hauptproteinquelle. Molkenprotein zeichnet sich durch einen hohen Leucingehalt und eine schnelle Verdaulichkeit aus. Während eine schnelle Verdaulichkeit von Molkenprotein für den Muskelaufbau als zielführend angesehen wird, erscheint eine Proteinquelle mit langsamerer Verdaulichkeit für das Ziel der anhaltenden Sättigung sinnvoller.

Der erhöhte Verzehr tierischen Proteins steht im Verdacht, zu verschiedenen Zivilisationskrankheiten beizutragen, während dieser Zusammenhang für einen erhöhten Verzehr pflanzlichen Proteins nicht beschrieben ist [Tharrey et al. Patterns of plant and animal protein intake are strongly associated with cardiovascular mortality. The Adventist Health Study-2 cohort. International Journal of Epidemiology, 2018].

Auf der anderen Seite gelten pflanzliche Proteine als unvollständig, da sie meist nicht alle essentiellen Aminosäuren enthalten. Von der WHO wurden in Zusammenarbeit mit der FAO Empfehlungen für die Aminosäurezusammensetzung eines "optimalen" Proteins veröffentlicht [WHO. Protein and Amino Acid Requirements in Human Nutrition, WHO Technical Report Series 935, 2007]. Eine Zusammensetzung, die eine Mischung aus Pflanzenproteinhydrolysat und Erbsenhydrolysat enthält, ist bereits bekannt aus den Dokumenten DATABASE GNPD MINTEL; 10. September 2015: "Carob Flavored Rice & Pea Protein",DATABASE GNPD [Online] MINTEL; 10. September 2015: "Carob Flavored Rice & Pea Protein" und US5547927 A (COPE FREDERICK O [US] ET AL).

Aufgabe der vorliegenden Erfindung ist es, eine Zusammensetzung, insbesondere zur Verwendung als Nahrungsergänzungsmittel, Lebensmittel des allgemeinen Verzehrs oder funktionelles Lebensmittel bereitzustellen, die gegenüber den bisher bekannten Zusammensetzungen, insbesondere gegenüber Präparaten auf Basis tierischer Proteine und/oder auf Basis rein pflanzlicher Proteine, verbesserte Eigenschaften aufweist.

Gelöst wird die Aufgabe durch eine Zusammensetzung, insbesondere zur Verwendung als Nahrungsergänzungsmittel, als Lebensmittel des allgemeinen Verzehrs oder als funktionelles Lebensmittel, enthaltend die Komponenten:
a) 30 bis 85 Gew.-% Erbsenproteinisolat,
b) 2 bis 30 Gew.-% Pflanzenprotein-Hydrolysat,
c) 2 bis 30 Gew.-% Kürbiskernprotein,
d) 5 bis 40 Gew.-% Haferextrakt,
e) 2 bis 20 Gew.-% Sanddornpulver, und
f) 0,1 bis 5 Gew.-% Acerolapulver.
Weitere bevorzugte Ausführungsformen ergeben sich aus den abhängigen Ansprüchen.

Die vorliegende Erfindung beruht demnach auf dem allgemeinen Gedanken, eine Zusammensetzung bereitzustellen, die ein pflanzliches Protein mit günstiger Aminosäurezusammensetzung enthält und dabei die Vorteile einer schnellen und einer langsamen Verdaulichkeit in sich vereinigt.

Überraschenderweise wurde gefunden, dass die erfindungsgemäße Zusammensetzung dem ernährungs- und altersbedingten Übergewicht und Muskelschwund entgegenwirkt.

Die erfindungsgemäße Zusammensetzung, insbesondere zur Verwendung als Nahrungsergänzungsmittel, als Lebensmittel des allgemeinen Verzehrs oder als funktionelles Lebensmittel, enthält die Komponenten:
a) 30 bis 85 Gew.-% Erbsenproteinisolat,
b) 2 bis 30 Gew.-% Pflanzenprotein-Hydrolysat,
c) 2 bis 30 Gew.-% Kürbiskernprotein,
d) 5 bis 40 Gew.-% Haferextrakt,
e) 2 bis 20 Gew.-% Sanddornpulver, und
f) 0,1 bis 5 Gew.-% Acerolapulver.

Ohne Bindung an diese Theorie ist davon auszugehen, dass die enthaltenen Peptide aus dem Pflanzenprotein-Hydrolysat schnell den Dünndarm erreichen und dort schnell in den Blutkreislauf gelangen und ein erstes Sättigungsgefühl hervorrufen.

Das Erbsenproteinisolat hingegen muss zunächst durch Verdauungsenzyme aufgespalten werden, bevor die enthaltenen Peptide und Aminosäuren resorbiert werden können. Somit ist eine anhaltende Zufuhr von Aminosäuren gewährleistet, die zum einen Sättigungseffekte hervorrufen und zum anderen wichtige Bausteine für den Muskelaufbau darstellen.

Durch die Kombination der Rohstoffe entspricht die Aminosäurezusammensetzung weitestgehend den Empfehlungen der WHO und FAO für eine optimale Proteinzusammensetzung. Besonders der Gehalt an verzweigtkettigen, d.h. an den proteinogenen Aminosäuren Valin, Leucin und Isoleucin, und an aromatischen Aminosäuren ist bemerkenswert. Der hohe Gehalt an den aromatischen Aminosäuren Phenylalanin und Tyrosin kann appetithemmende und auch langanhaltende stimmungsaufhellende Wirkungen entfalten.

Auch L-Arginin und L-Lysin kommen in großen Mengen vor und können daher positive Effekte für eine optimale Stoffwechselsteuerung mit Stimulation der Fettverbrennung auslösen. Hierzu bedarf es vor allem des L-Arginins, das die Bildung von Stickstoffmonoxid, NO, einem entscheidenden Stimulator der Fettverbrennung fördert. L-Leucin und die beiden anderen verzweigtkettigen Aminosäuren L-Isoleucin und L-Valin werden ebenfalls in ausreichenden Mengen und im bevorzugten Verhältnis 2:1:1 zugeführt, um einen dauerhaften Muskelaufbau und Muskelerhalt mit verstärkter mitochondrialer metabolischer Aktivität sicherzustellen.

Der Gehalt an Methionin ist bewusst gering gewählt, weil diese schwefelhaltige Aminosäure im Verdacht steht, die nachteiligen Effekte des erhöhten Verzehrs tierischen Proteins mit zu verursachen.

Die enthaltenen Ballaststoffe wirken sich günstig auf die Verdauung aus, insbesondere die enthaltenen β-Glucane aus Hafer. Vitamin C trägt zu einem normalen Energiestoffwechsel bei. Zink trägt zu einer normalen Eiweißsynthese und zu einem normalen Stoffwechsel von Makronährstoffen wie Kohlenhydraten und Fettsäuren bei.

Mit der vorliegenden Erfindung wird somit eine Stoffwechselsteuerung durch eine zielführende Nährstoffergänzung angestrebt.

Das Erbsenproteinisolat, ein natürliches, rein pflanzliches Protein, der Komponente a) hat einen Proteingehalt von mindestens 60 %, vorzugsweise über 80 %, und beinhaltet sämtliche der neun essentiellen Aminosäuren. Das Pflanzenprotein-Hydrolysat hat einen Proteingehalt von mindestens 75 % und wird vorzugsweise aus Erbsen *(Pisum sativum)* gewonnen. Die Herstellung erfolgt bevorzugt mittels enzymatischer Hydrolyse.

Gemäß einer bevorzugten Variante enthält das Hydrolysat eine Molekulargewichtsverteilung, in der mindestens 25 % der Peptide ein Molekulargewicht < 1000 Da und mindestens 50 % der Peptide ein Molekulargewicht < 5000 Da aufweisen.

Vorzugsweise sind in der Zusammensetzung a) Erbsenproteinisolat und b) Pflanzenprotein-Hydrolysat bevorzugt im Gewichtsverhältnis von 10:1 vorhanden.

Das Kürbiskernprotein c) hat in einer bevorzugten Ausführungsform einen Proteingehalt von > 50 %, und dient zudem als Quelle der Mineralstoffe Zink und Eisen.

Der Haferextrakt d) wird in einer bevorzugten Ausführungsform in Form von Haferkleie mit einem β-Glucangehalt von 5 bis 30 % und einem Proteingehalt von 15 bis 30 %, besonders bevorzugt mit einem β-Glucangehalt von 7 bis 10 % und einem Proteingehalt von 20 bis 25 % eingesetzt.

Das Sanddornpulver e) wird in einer bevorzugten Ausführungsform aus Sanddornfeinmark hergestellt, indem es mit Hilfe von Maltodextrin sprühgetrocknet wird. Sanddornpulver kann in einer besonders bevorzugten Form auch als Vitamin C-Quelle dienen.

Das Acerolapulver f) wird in einer bevorzugten Ausführungsform als Acerola-Saftkonzentrat hergestellt, das mit Hilfe von Gummi arabicum sprühgetrocknet wird.

Neben den Komponenten a) bis f) kann die Zusammensetzung in einer besonders bevorzugten Ausführungsform weitere Komponenten enthalten, ausgewählt aus einer Gruppe, zumindest umfassend, vorzugsweise natürliche Aromen, Aromaextrakten, Süßungsmitteln, Fließregulierungsmitteln und/oder Trennmitteln.

Diese sind vorzugsweise alle für die Verwendung in Lebensmitteln zugelassen (vgl. Verordnung EU 1129/2011 bzw. Zusatzstoff-Zulassungsverordnung). In einer weiteren besonders bevorzugten Ausführungsform werden nur pflanzliche Rohstoffe aus kontrolliert biologischem Anbau verwendet. Die genannten weiteren Komponenten entsprechen in diesem Fall der Öko-VO (EU) 834/2007 und ihren Erweiterungen.

Als natürliche Aromen und/oder Aroma-Extrakte werden insbesondere Fruchtextrakte und Fruchtaromen eingesetzt. Als Früchte werden insbesondere Sanddorn, Pfirsich, Maracuja, Passionsfrucht, Brombeere, Papaya und/oder Mango verwendet. In einer besonders bevorzugten Ausführungsform wird Brombeerblattextrakt eingesetzt.

Als Süßungsmittel werden synthetische Süßsstoffe wie Sucralose, Aspartam, Acesulfam K, Natrium-Cyclamat, Saccharin-Natrium, Steviol-Glykoside oder Thaumatin bzw. Zuckeralkohole wie Erythrit, Xylit, Isomalt oder Sorbit verwendet. In einer besonders bevorzugten Ausführungsform wird biologisch zertifiziertes Erythrit angewendet.

Als Fließregulierungs- und Trennmittel kommen insbesondere Tri-Calciumphosphat, Magnesiumsalze von Speisefettsäuren (Magnesiumstearat), Siliciumdioxid oder Reishüllen-Extrakt infrage.

In einer bevorzugten Ausführungsform, in der alle Komponenten a) bis f) enthalten sind, können diese in den folgenden Konzentrationen gemäß Tabelle 1 vorhanden sein, wobei der Rest zu 100 % ggf. weitere Komponenten aus der obigen Aufzählung sind.

**Tabelle 1**

| Komponente | | Menge in Gew.-% |
|---|---|---|
| | a) Erbsenprotein-Isolat | 30 bis 85 |
| | | vorzugsweise 50 bis 60 |
| | b) Pflanzenprotein-Hydrolysat | 2 bis 30 |
| | | vorzugsweise 5 bis 10 |
| | c) Kürbiskernprotein | 2 bis 30 |
| | | Vorzugsweise 5 bis 10 |
| | d) Haferextrakt | 5 bis 40 |
| | | vorzugsweise 10 bis 20 |
| | e) Sanddornpulver | 2 bis 20 |
| | | vorzugsweise 5 bis 10 |
| | f) Acerolapulver | 0,1 bis 5 |
| | | vorzugsweise 0,2 bis 1,5 |

In einer weiteren bevorzugten Ausführungsform sind maximal die Komponenten a) bis f) und Aromaextrakt, insbesondere als natürliches Aroma, enthalten, aber keine weiteren Inhaltsstoffe, d.h. die Zusammensetzung besteht in dieser Ausführungsform maximal aus den genannten Bestandteilen.

Die erfindungsgemäße Zusammensetzung kann in vorteilhafterweise mit weiteren Wirkstoffen kombiniert werden, die eine Proteinquelle darstellen oder eine verdauungsfördernde oder antioxidative Wirkung haben, d.h. die Zusammensetzung kann weitere Wirkstoffe aufweisen. Solche Wirkstoffe sind bevorzugt Proteinquellen wie Molke, Hefe, Sojaprotein, Casein, Milchprotein, Hanfprotein, Lupinenprotein, Mungobohnenprotein, Kollagen oder Proteinisolate weiterer Hülsenfrüchte, weitere Ballaststoffquellen wie Haferfaser, Inulin, Fructo-Oligosaccharide, Guar, Gerste oder weitere vitaminreiche Fruchtextrakte aus Baobab oder Camu-Camu.

In einer weiteren bevorzugten Ausführungsform besteht die Zusammensetzung maximal aus den Komponenten a) bis f), Aroma, Süßungsmittel, Fließregulierungs- und Trennmittel und einem oder mehreren der im vorangehenden Absatz abschließend aufgezählten weiteren Wirkstoffe, es sind keine weiteren Inhaltsstoffe vorhanden.

Die Formulierung "besteht maximal aus" bedeutet, dass eine oder mehrere der genannten Bestandteile - abgesehen von a) und b) - auch fehlen können, aber außer den genannten Bestandteilen keine weiteren Inhaltsstoffe enthalten sind. Vorzugsweise sind alle maximal möglichen genannten Bestandteile enthalten.

Die erfindungsgemäße Zusammensetzung wird insbesondere als Nahrungsergänzungsmittel verwendet, wobei die Art der Verwendung nicht begrenzt ist, d.h. insbesondere jede Art von oraler Applikation ist möglich, vorzugsweise werden allerdings Pulver oder Granulate zum Herstellen einer Suspension oder Lösung eingesetzt.

Diese Applikationsform erleichtert die Einnahme durch den Anwender. Die Verzehrempfehlung beträgt 1-2 Portionen täglich, bevorzugt vor dem Frühstück und/oder nach dem Abendessen. Das Produkt wird dazu in einem Glas stillem Wasser, Saft oder Milch (vorzugsweise ad 200 ml) eingerührt und dann getrunken. Das Produkt soll über einen längeren Zeitraum von mindestens 3-6 Monaten eingenommen werden und kann dann alle erwünschten ernährungsphysiologischen Wirkungen erfüllen.

Die überragende Wirksamkeit der erfindungsgemäßen Zusammensetzung im Hinblick auf ernährungs- und altersbedingtes Übergewicht und Muskelschwund konnte in einer Studie nachgewiesen werden.

Eine produktspezifische klinische Pilotstudie an 132 älteren Probanden ergab nach 6 Monaten Einnahme einer Portion pro Tag eine Gewichtsreduktion von 3.9 ± 0.3 kg, eine Reduktion der Fettmasse von 6.4 kg ± 0.7 kg und eine Zunahme der Muskelmasse von 2.5 kg ± 0.3 kg. Bei Einnahme von zwei Portionen pro Tag betrug die Gewichtsreduktion bereits 7.7 kg ± 0.8 kg, die Reduktion der Fettmasse 12.7 ± 1.4 kg und die Zunahme der Muskelmasse 5.0 ± 0.5 kg.

Damit konnte ein erster Nachweis für die einzigartigen synergistischen ernährungsphysiologischen Wirkungen dieser Nährstoffsupplementation erbracht werden. Auch Stoffwechselstörungen wie erhöhte Glucose, Insulin und HbA1c Werte konnten durch die Einnahme des Nahrungsergänzungsmittels normalisiert werden. Damit zeichnet sich ein neuer Ansatz für eine dauerhafte Verringerung des Übergewichts ab, der gleichzeitig dem Muskelschwund entgegenwirkt und Stoffwechselstörungen abstellen kann. Solche Wirkungen konnten nicht nach einer Placebo-Gabe (1 oder 2 x Milcheiweiß mit Orangenaroma) erzielt werden. Sie gehen damit von den endogenen Nährstoffen und Naturwirkstoffen in dem Gesundheitsprodukt, d. h. der erfindungsgemäßen Zusammensetzung, aus. Therapeutische bzw. medizinische Anwendungen der erfindungsgemäßen Zusammensetzung sind dabei explizit ausgenommen.

Gegebenenfalls könnten demnach als Ergebnis oben genannter Studie für die erfindungsgemäße Zusammensetzung folgende Gesundheitsaussagen gemacht werden:
- Proteine tragen zu einer Zunahme an Muskelmasse bei
- Proteine tragen zur Erhaltung von Muskelmasse bei
- Vitamin C trägt zu einem normalen Energiestoffwechsel bei
- Vitamin C trägt dazu bei, die Zellen vor oxidativem Stress zu schützen
- Zink trägt zu einem normalen Stoffwechsel von Makronährstoffen bei
- Zink trägt zu einem normalen Kohlenhydrat-Stoffwechsel bei

Die erfindungsgemäße Zusammensetzung ist geeignet, insbesondere bei einem gesunden Menschen, insbesondere unter Verwendung als Nahrungsergänzungsmittel, als Lebensmittel des allgemeinen Verzehrs oder als funktionelles Lebensmittel einem ernährungs- und altersbedingten Übergewicht und Muskelschwund entgegenzuwirken.

## Patentansprüche

1. Zusammensetzung, insbesondere zur Verwendung als Nahrungsergänzungsmittel, als Lebensmittel des allgemeinen Verzehrs oder als funktionelles Lebensmittel, enthaltend die Komponenten
a) 30 bis 85 Gew.-% Erbsenproteinisolat,
b) 2 bis 30 Gew.-% Pflanzenprotein-Hydrolysat,
c) 2 bis 30 Gew.-% Kürbiskernprotein,
d) 5 bis 40 Gew.-% Haferextrakt,
e) 2 bis 20 Gew.-% Sanddornpulver, und
f) 0,1 bis 5 Gew.-% Acerolapulver.

2. Zusammensetzung gemäß Anspruch 1, enthaltend die Komponenten
a) 50 bis 60 Gew.-% Erbsenproteinisolat,
b) 5 bis 10 Gew.-% Pflanzenprotein-Hydrolysat,
c) 5 bis 10 Gew.-% Kürbiskernprotein,
d) 10 bis 20 Gew.-% Haferextrakt,
e) 5 bis 10 Gew.-% Sanddornpulver, und
f) 0,2 bis 1,5 Gew.-% Acerolapulver.

3. Zusammensetzung nach Anspruch 1 oder 2, **dadurch gekennzeichnet, dass** weitere Komponenten enthalten sind, ausgewählt aus einer Gruppe, zumindest umfassend Aromen, Aroma-Extrakte, Süßungsmittel, Fließregulierungs- und/oder Trennmittel.

4. Zusammensetzung nach einem oder mehreren der Ansprüche 1-3, **dadurch gekennzeichnet, dass** das Pflanzenprotein-Hydrolysat aus Erbse *(Pisum sativum)* gewonnen wird.

5. Zusammensetzung nach einem oder mehreren der Ansprüche 1-4, **dadurch gekennzeichnet, dass** a) Erbsenproteinisolat und b) Pflanzenproteinhydrolysat im Gewichtsverhältnis von 10:1 vorhanden sind.

6. Zusammensetzung nach einem oder mehreren der Ansprüche 1-5, **dadurch gekennzeichnet, dass** der Haferextrakt d) in Form von Haferkleie mit einem β-Glucangehalt von 5 bis 30 % und einem Proteingehalt von 15 bis 30 % eingesetzt wird.

7. Zusammensetzung nach einem oder mehreren der vorangegangenen Ansprüche, die ferner einen oder mehrere aktive(n) Inhaltsstoff(e), ausgewählt aus Proteinquellen wie Molke, Hefe, Sojaprotein, Casein, Milchprotein, Hanfprotein, Lupinenprotein, Mungobohnenprotein oder Proteinisolaten weiterer Hülsenfrüchte, weiteren Ballaststoffquellen wie Haferfaser, Inulin, Fructo-Oligosaccharide, Guar, Gerste oder weiteren vitaminreichen Fruchtextrakten, enthält.

8. Zusammensetzung nach einem oder mehreren der Ansprüche 1-7, die als Pulver, Granulat, Tablette, Kapsel, Suspension, Emulsion, Lösung, Kautablette oder Riegel vorliegt.

9. Zusammensetzung nach einem oder mehreren der Ansprüche 1 bis 8 zur Verwendung zur Nahrungsergänzung, zum Gewichtsmanagement, zum Erhalt und/oder Aufbau der Muskelmasse und/oder zur Stoffwechselsteuerung.

## Claims

1. Composition, in particular for use as a food supplement, as a food for general consumption or as a functional food, containing the components
a) 30 to 85 wt.-% pea protein isolate,
b) 2 to 30 wt.-% plant protein hydrolyzate,
c) 2 to 30 wt.-% pumpkin seed protein,
d) 5 to 40 wt.-% oat extract,
e) 2 to 20 wt.-% sea buckthorn powder, and
f) 0.1 to 5 wt.-% acerola powder.

2. Composition according to claim 1, containing the components
a) 50 to 60 wt.-% pea protein isolate,
b) 5 to 10 wt.-% plant protein hydrolyzate,
c) 5 to 10 wt.-% pumpkin seed protein,
d) 10 to 20 wt.-% oat extract,
e) 5 to 10 wt.-% sea buckthorn powder, and
f) 0.2 to 1.5 wt.-% acerola powder.

3. Composition according to claim 1 or 2, **characterized in that** further components are contained, selected from a group comprising at least flavors, flavor extracts, sweeteners, flow regulators and/or anti-caking agents.

4. Composition according to one or more of claims 1-3, **characterized in that** the plant protein hydrolyzate is obtained from pea *(Pisum sativum).*

5. Composition according to one or more of claims 1-4, **characterized in that** a) pea protein isolate and b) plant protein hydrolyzate are present in a weight ratio of 10:1.

6. Composition according to one or more of claims 1-5, **characterized in that** the oat extract d) is used in the form of oat bran with a β-glucan content of 5 to 30% and a protein content of 15 to 30%.

7. Composition according to one or more of the preceding claims, further containing one or more active ingredient(s) selected from protein sources such as whey, yeast, soy protein, casein, milk protein, hemp protein, lupin protein, mung bean protein or protein isolates of further legumes, further fiber sources such as oat fiber, inulin, fructo-oligosaccharides, guar, barley or further vitamin-rich fruit extracts.

8. Composition according to one or more of claims 1-7, which is present as powder, granules, tablet, capsule, suspension, emulsion, solution, chewable tablet or bar.

9. Composition according to one or more of claims 1 to 8 for use in nutritional supplementation, weight management, maintaining and/or building muscle mass and/or for metabolic control.

## Revendications

1. Composition, en particulier pour l'utilisation en tant que complément alimentaire, en tant qu'aliment de consommation générale ou en tant qu'aliment fonctionnel, contenant les composants
a) 30 à 85 % en poids d'isolat de protéines de pois,
b) 2 à 30 % en poids d'hydrolysat de protéines végétales,
c) 2 à 30 % en poids de protéines de pépins de courge,
d) 5 à 40 % en poids d'extrait d'avoine,
e) 2 à 20 % en poids de poudre d'argousier, et
f) 0,1 à 5 % en poids de poudre d'acérola.

2. Composition selon la revendication 1, contenant les composants
a) 50 à 60 % en poids d'isolat de protéines de pois,
b) 5 à 10 % en poids d'hydrolysat de protéines végétales,
c) 5 à 10 % en poids de protéines de pépins de courge,
d) 10 à 20 % en poids d'extrait d'avoine,
e) 5 à 10 % en poids de poudre d'argousier, et
f) 0,2 à 1,5 % en poids de poudre d'acérola.

3. Composition selon la revendication 1 ou 2, **caractérisée en ce que** des composants supplémentaires sont contenus, sélectionnés parmi un groupe comprenant au moins des arômes, des extraits d'arômes, des édulcorants, des agents de régulation d'écoulement et/ou de séparation.

4. Composition selon une ou plusieurs des revendications 1-3, **caractérisée en ce que** l'hydrolysat de protéines végétales est obtenu à partir de pois *(Pisum sativum).*

5. Composition selon une ou plusieurs des revendications 1-4, **caractérisée en ce que** a) l'isolat de protéines de pois et b) l'hydrolysat de protéines végétales sont présents dans un rapport pondéral de 10:1.

6. Composition selon une ou plusieurs des revendications 1-5, **caractérisée en ce que** l'extrait d'avoine d) est utilisé sous forme de son d'avoine avec une teneur en β-glucane de 5 à 30 % et une teneur en protéines de 15 à 30 %.

7. Composition selon une ou plusieurs des revendications précédentes, qui contient en outre un ou plusieurs ingrédient(s) actif(s) sélectionné(s) parmi des sources de protéines telles que le lactosérum, la levure, la protéine de soja, la caséine, la protéine de lait, la protéine de chanvre, la protéine de lupin, la protéine de haricot mungo ou les isolats de protéines de légumineuses supplémentaires, des sources de fibres alimentaires supplémentaires telles que les fibres d'avoine, l'inuline, les fructo-oligosaccharides, le guar, l'orge ou des extraits de fruits riches en vitamines supplémentaires.

8. Composition selon l'une ou plusieurs des revendications 1-7, qui se présente sous forme de poudre, de granulés, de comprimés, de gélules, de suspension, d'émulsion, de solution, de comprimés à croquer ou de barre.

9. Composition selon une ou plusieurs des revendications 1 à 8, destinée à être utilisée comme complément alimentaire, pour la gestion du poids, pour le maintien et/ou le développement de la masse musculaire et/ou pour le contrôle du métabolisme.
